# EUROPEAN PATENT APPLICATION

(11) **EP 4 834 330 A1**
(43) Date of publication of application: **04.11.2026**
(21) Application number: 24913438.8
(22) Date of filing: 27.12.2024
(51) Int. Cl.: A61K 9/16, A61K 9/00, A61K 31/351, A61K 38/26, A61P 3/04

(54) **INJECTABLE DEPOT COMPOSITION COMPRISING ENAVOGLIFLOZIN AND LOW-DOSE LIRAGLUTIDE**

(30) Priority: 28.12.2023 KR 20230195363
(71) Applicant: Daewoong Pharmaceutical Co., Ltd., Hwaseong-si, Gyenoggi-do 18623 (KR)
(72) Inventor: KIM, Gwan Young, Yongin-si Gyeonggi-do 17028 (KR); KIM, Jun Sik, Yongin-si Gyeonggi-do 17028 (KR); BAE, Byoung Chan, Yongin-si Gyeonggi-do 17028 (KR); IM, Byeong Nam, Yongin-si Gyeonggi-do 17028 (KR)
(74) Representative: Plasseraud IP
(86) International application number: PCT/KR2024/021203
(87) International publication number: WO 2025/143831

(57) **Abstract**

The present invention provides an injectable depot composition comprising enavogliflozin and a low-dose liraglutide. A combination regimen of enavogliflozin and a low-dose liraglutide provides a synergistic effect in the treatment of obesity compared to the sole administration of either of the drugs. In addition, compared to the sole administration of liraglutide, the administered dose of liraglutide can be reduced, and thus direct causes of gastrointestinal and cardiovascular side effects of liraglutide can be mitigated. In addition, the present invention has the advantage of additionally providing the effect of alleviating metabolic diseases and cardiovascular diseases related to blood glucose, blood lipids, blood pressure, fatty liver, and the like through combined administration with enavogliflozin. Furthermore, the depot composition of enavogliflozin and liraglutide according to the present invention can significantly improve medication convenience and medication adherence for a patient compared to existing liraglutide formulations, which are subcutaneously injected once a day, by unifying the route of administration and improving the administration cycle.

## Description

### [Technical Field]

The present invention relates to an Injectable depot composition comprising enavogliflozin and low-dose liraglutide.

### [Background Art]

A GLP-1 receptor agonist (GLP-1 Receptor Agonist, hereinafter also referred to as GLP-1RA) is a drug that activates GLP-1 receptors to reduce blood glucose and energy absorption. The GLP-1 receptor agonist mimics the action of GLP-1, an endogenous incretin hormone secreted from the intestine after a meal. GLP-1 receptor agonists inhibit glucagon secretion, stimulate insulin production to reduce hyperglycemia in patients with type 2 diabetes, and are effective in treating obesity through weight loss by reducing food intake. The inhibition of glucagon secretion by GLP-1 receptor agonists occurs in proportion to glucose concentration. That is, it has the advantage of not causing serious problems in a hypoglycemic situation because glucagon secretion is inhibited at high blood glucose and glucagon secretion is not inhibited at low blood glucose. In the case of liraglutide, a GLP-1RA drug well known under the trade name Saxenda, it was developed as a therapeutic agent for diabetes in the early stage of development, but it is known to be effective against obesity when administered at a high dose, and is currently widely used for the treatment of obesity. However, the prescribed dose of liraglutide for obesity reaches 3 to 5 times the prescribed dose for diabetes, and unlike when administering the prescribed dose for diabetes, it is known that when administering the prescribed dose for obesity, there are a number of side effects, including gastrointestinal side effects such as nausea and diarrhea, and cardiovascular side effects such as an increased heart rate due to the high-dose prescription.

Meanwhile, SGLT-2 inhibitors are drugs having a mechanism of weight reduction and blood pressure reduction effects through osmotic diuresis and caloric loss caused by glucose excretion. Enavogliflozin, an SGLT-2 inhibitor developed by the present applicant, is a drug recently approved for marketing as an oral therapeutic agent for diabetes. According to a prior study of the present applicant, it was confirmed that enavogliflozin is excellent in obesity treatment effect based on the ability to inhibit glucose reabsorption.

Accordingly, the present inventors conducted research under the hypothesis that the combination administration of enavogliflozin and low-dose liraglutide would cause synergy in the treatment of obesity, thereby improving the anti-obesity effect while reducing side effects compared to conventional therapeutic agents. As a result, they confirmed that the combination administration of enavogliflozin and low-dose liraglutide reduces the side effects of liraglutide and exhibits a synergistic effect in weight reduction compared to the single administration of the two drugs, and furthermore, confirmed that a long-acting combination formulation can be provided by preparing these drugs into an injectable depot composition, thereby completing the present invention.

### [Disclosure]

### [Technical Problem]

An object of the present invention is to provide a novel anti-obesity pharmaceutical composition capable of improving an anti-obesity effect while reducing side effects due to high-dose administration of liraglutide, and simultaneously improving durability of efficacy, patient medication convenience, and medication compliance.

### [Technical Solution]

In the course of conducting research to reduce side effects due to high-dose administration of liraglutide, the present inventors confirmed that combination administration of enavogliflozin (oral administration once a day) and low-dose liraglutide (subcutaneous injection once a day) reduces the side effects of liraglutide, while exhibiting a synergistic effect in body weight reduction compared to single administration of the two drugs. A difference in the administration route of each drug does not affect the achievement of an enhanced anti-obesity effect, but unification of the administration route may contribute to improving patient medication convenience and medication compliance, and thus it is preferable to administer the two drugs through a single administration route.

In addition, taking a medication once a day, particularly in the case of an injection, not only causes aversion to injection in patients but is also very inconvenient. Anti-obesity treatment is usually performed for a long period of 2 weeks, 4 weeks, or more, and subcutaneous injection once a day may be a factor that significantly reduces patient medication convenience and medication compliance, so improvement of such a dosing regimen of subcutaneous injection once a day was also a problem that the present inventors intended to solve.

Accordingly, the present inventors conducted continuous research to develop a combination formulation for unification of the administration route and dosing regimen for the combination administration of enavogliflozin and low-dose liraglutide. As a method capable of avoiding subcutaneous injection once a day, the present inventors conceived a depot composition developable as a sustained-release injection. As a result, it was confirmed that preparing an injectable depot composition comprising enavogliflozin and low-dose liraglutide as active ingredients may improve sustained efficacy, patient medication convenience, and medication compliance.

Accordingly, the present invention provides an injectable depot composition comprising microparticles comprising enavogliflozin and liraglutide as active ingredients, and a biodegradable polymer.

A depot composition comprising microparticles comprising a drug and a biodegradable polymer may be utilized as a sustained-release injection that exhibits long-term efficacy as the drug is slowly released during a decomposition process of the microparticles.

In the depot composition, a method of encapsulating a plurality of drugs within a single microparticle, or separately encapsulating two or more types of drugs into two or more types of microparticles may also be used, and thus it is possible to formulate a plurality of drugs into a single dosage form.

However, since enavogliflozin is a small molecule drug and liraglutide is a peptide drug, it is actually not easy to formulate drugs having different properties into a single dosage form. In the case of enavogliflozin, since it is a small molecule drug and has low water solubility (1 mg/mL or less), use of an organic solvent is inevitable for manufacturing a microparticle. On the other hand, since liraglutide is a peptide drug and has excellent water solubility (about 50 mg/mL), it is not dissolved at all in a volatile organic solvent (for example, methylene chloride) essentially used for manufacturing a microparticle. As such, there is a significant difference in physicochemical properties between the two drugs, and thus there is significant difficulty throughout a manufacturing process of a microparticle, a drug encapsulation process, and drug release control overall. In order to solve this problem, the present inventors attempted to control an amount of an organic solvent used to dissolve enavogliflozin and various manufacturing processes affecting encapsulation of liraglutide (control of a first emulsification condition, control of a second emulsification condition, control of a solvent evaporation process, an additive, and control of solubility through salt modification of liraglutide). As a result, they demonstrated that it is possible to manufacture a combination depot composition in which two types of drugs can be released at a constant effective concentration for a target period.

In the pharmaceutical composition of the present invention, enavogliflozin or liraglutide includes all cases where it exists in the form of a pharmaceutically acceptable salt, a pharmaceutically acceptable amide, or a pharmaceutically acceptable ester thereof. For example, in an embodiment of the present invention, liraglutide may exist as liraglutide acetate.

A manufacturing technology of a microparticle comprising a drug and a biodegradable polymer is well known in the art.

For example, the biodegradable polymer may be selected from the group consisting of polylactide (PLA), polyglycolide (PGA), poly(lactide-co-glycolide) (PLGA), and a mixture thereof. A ratio of polylactide to polyglycolide in the poly(lactide-co-glycolide) copolymer may be 50:50 to 95:5, for example, 50:50, 60:40, 65:35, 70:30, 75:25, 80:20, or 85:15.

Without being limited thereto, the biodegradable polymer may have a weight average molecular weight of 4,000 to 50,000. For example, the weight average molecular weight of the biodegradable polymer includes all sub-numerical ranges within the range, such as a weight average molecular weight of 4,000 to 15,000, a weight average molecular weight of 7,000 to 17,000, a weight average molecular weight of 5,000 to 20,000, a weight average molecular weight of 8,000 to 30,000, a weight average molecular weight of 8,000 to 20,000, a weight average molecular weight of 10,000 to 18,000, and a weight average molecular weight of 18,000 to 28,000.

For example, as the biodegradable polymer used in the present invention, polylactide, polyglycolide, or poly(lactide-co-glycolide) under a trade name of RESOMER^{™} of Evonik Rohm GmbH may be used, or a blend thereof may be used. For instance, R202H, R202S, R203H, R203S, RG502H, RG503H, RG653H, RG752H, RG752S, RG753H, and RG753S may be used alone or as a blend.

A suitable molecular weight or a blending ratio of the biodegradable polymer may be appropriately selected by a person skilled in the art in consideration of a decomposition rate of the biodegradable polymer and a drug release rate accordingly. Without being limited thereto, in an embodiment, the biodegradable polymer may be a polylactide having a weight average molecular weight of 8,000 to 30,000.

In an embodiment of the present invention, the microparticle may be prepared by a solvent evaporation method or a spray drying method.

The solvent evaporation method refers to, for example, a method of preparing an O/W or W/O/W emulsion and then evaporating a solvent therefrom to form a microparticle. An emulsion is obtained by mixing a mixed solution of a biodegradable polymer and a drug dissolved in a solvent with an aqueous medium, and the solvent is evaporated from the emulsion to form a microparticle containing the drug.

The spray drying method is a method of spraying a liquid phase comprising an active ingredient through a nozzle at a particular speed and simultaneously drying with hot air to obtain a powder form. A mixed solution prepared by dissolving a biodegradable polymer and a drug in an organic solvent is sprayed in a liquid phase through a nozzle and simultaneously dried to recover a microparticle of a particular size containing the drug.

For example, the microparticle may be a W/O/W type microparticle prepared by evaporating a solvent after preparing a W/O/W emulsion.

Encapsulation of a drug within a microparticle may vary depending on a manufacturing method. As described above, since liraglutide is water-soluble while enavogliflozin is poorly water-soluble, there is difficulty in utilizing an O/W method in which a solvent capable of simultaneously dissolving a plurality of drugs is used to mix with an organic solvent dissolving a biodegradable polymer. This is because a co-solvent capable of simultaneously dissolving both drugs in an organic solvent and an aqueous phase could not be found due to a difference in solubility between enavogliflozin and liraglutide. Accordingly, in an example of the present invention, a microparticle was prepared by using a W/O/W manufacturing method in which liraglutide is dissolved in an aqueous phase, a W/O emulsion is prepared by mixing the aqueous phase with an organic solvent comprising a biodegradable polymer utilizing a variety of organic solvents capable of dissolving enavogliflozin, and the W/O emulsion containing the liraglutide of the aqueous phase is remixed with a second aqueous phase.

In the case of a W/O/W type microparticle, a W/O emulsion is prepared by utilizing a homomixer on an oil phase solution and a first aqueous phase solution, and then this is mixed again with a second aqueous phase solution to form a W/O/W type microparticle. At this time, the first aqueous phase solution may be water, for example, primary distilled water. The first aqueous phase solution may also comprise a PBS buffer. Without being limited thereto, an oil phase solvent may be methylene chloride, acetic acid, or the like. The oil phase solution may comprise a biodegradable polymer which is polylactide, polyglycolide, poly(lactide-co-glycolide), or a blend thereof. An organic solvent capable of dissolving enavogliflozin may comprise methanol, dimethyl sulfoxide, acetonitrile, n-methyl-2-pyrrolidone, or the like. The second aqueous phase solution may be a surfactant aqueous solution. As a surfactant used in the second aqueous phase solution, polyvinyl alcohol may be used.

A W/O emulsion may be prepared by mixing an oil phase solution and a first aqueous phase solution and homogenizing by stirring utilizing a homogenizer. Specifically, a W/O emulsion may be prepared by mixing a first aqueous phase solution(W) comprising liraglutide with an oil phase solution(O) which is a mixture of a solution in which enavogliflozin is dissolved in an organic solvent and a solution in which a polymer is dissolved in an organic solvent.

A W/O/W type emulsion may be prepared by introducing the prepared W/O emulsion again into a second aqueous phase solution comprising a surfactant. At this time, similarly, an emulsion may be prepared by homogenizing through stirring utilizing a homogenizer. A stirring speed and time for homogenization are not limited thereto, but generally, stirring may be performed at 500 to 20,000 rpm for 1 minute to 5 minutes. Solvent evaporation is performed on the homogenized emulsion, and the evaporation of the solvent may be performed while stirring at 50 to 600 RPM at a temperature of 5 to 40 °C for 1 hour to 4 hours.

In the present invention, a variety of organic solvents were searched to improve a drug encapsulation efficiency of enavogliflozin in consideration of organic solvent solubility, and among them, methanol was mainly used, which is more easily removable during a solvent evaporation process and a freeze-drying process compared to another organic solvent and thus leaves a small amount of a solvent remaining in a microparticle. In addition, in order to increase the drug encapsulation efficiency of enavogliflozin, an optimal concentration range of enavogliflozin and an amount of an organic solvent were established by searching for an appropriate ratio at a level where enavogliflozin is not precipitated by methylene chloride used as an oil phase co-solvent, and conversely, a biodegradable polymer dissolved in methylene chloride is not precipitated due to the organic solvent used to dissolve enavogliflozin. Without being limited thereto, a concentration of enavogliflozin in the organic solvent may be 10 to 20 w/w%. In addition, a weight ratio of an oil phase solvent (for example, methylene chloride) to a solvent for enavogliflozin (for example, methanol) may preferably be 4 to 6:1.

In an embodiment of the present invention, when preparing a W/O/W type microparticle, sodium acetate or magnesium hydroxide may be included as an additive during preparation of a W/O emulsion. Utilizing a W/O/W method, a manufacturing process capable of increasing a drug encapsulation efficiency of liraglutide within a microparticle was secured by adding sodium acetate to a first aqueous solution to adjust pH, and controlling stability and solubility of liraglutide, which is a peptide drug, in an aqueous solution phase. In another embodiment, magnesium hydroxide was added as an additive to induce even mixing with a biodegradable polymer during emulsion preparation to enable physical and chemical bonding with liraglutide, thereby increasing a drug encapsulation efficiency, suppressing an initial burst, and providing an effect of sustaining drug release for a long period compared to a liraglutide reference drug administered once a day.

In another example of the present invention, the microparticle may be prepared by preparing an S/O/W emulsion and then evaporating a solvent therefrom. For example, within the microparticle, liraglutide may exist in a state of solid liraglutide, for example, a state of liraglutide acetate. In the case of a microparticle comprising liraglutide, an initial burst may be problematic, and in this case, a side effect may be exhibited in a patient. In order to control the initial burst of the microparticle in which such liraglutide is encapsulated, in an example of the present invention, liraglutide was prepared and used as liraglutide acetate utilizing hydrophobic ion pairing. Hydrophobic ion pairing is a method in which a charged hydrophilic molecule is electrostatically bonded with an oppositely charged hydrophobic counterion to produce a complex having greater hydrophobicity than that of an original molecule. In the case of liraglutide acetate, it exhibits greater hydrophobicity at a particular pH of an aqueous phase and thus precipitates into a solid state. Utilizing such a property, liraglutide acetate can be recovered in a solid phase and utilized for preparing a microparticle in an S/O/W method. In addition, it was confirmed that in the case of such a solid-phase liraglutide acetate, it exhibits a reduced initial burst in vivo, is more stably encapsulated within a microparticle, and thus has potential to be more suitable for exhibiting drug release at a constant level or higher in a bodily environment.

In the case of an S/O/W type microparticle, a solid dispersion S/O is prepared by mixing an oil phase solution and solid-phase liraglutide acetate using a homomixer for several minutes, and then mixed into a second aqueous phase solution comprising a surfactant using a homogenizer to finally form the S/O/W type microparticle. An S/O emulsion may be prepared by homogenizing through stirring and mixing an oil phase solution and solid-phase liraglutide acetate using a homomixer. A stirring speed and time for homogenization are not limited thereto, but generally, homogenization may be performed at 500 to 25,000 rpm for 30 seconds to 3 minutes. At this time, a particle size of the solid-phase liraglutide acetate may be 0.1 to 10 µm, for example, 0.1 to 5 µm. Without being limited thereto, an oil phase solvent may be methylene chloride. The oil phase solution may comprise a biodegradable polymer selected from the group consisting of polylactide, polyglycolide, poly(lactide-co-glycolide), and a blend thereof. In addition, the oil phase solvent may comprise acetic acid, and may comprise methanol, dimethyl sulfoxide, acetonitrile, or n-methyl-2-pyrrolidone, which is an organic solvent capable of dissolving enavogliflozin. The second aqueous phase solution may be a surfactant aqueous solution. The second aqueous phase solution may also comprise polyvinyl alcohol. An S/O/W type emulsion may be prepared by introducing the prepared S/O emulsion into the second aqueous phase solution. At this time, similarly, an emulsion may be prepared by homogenizing through stirring using a homogenizer. A stirring speed and time for homogenization are not limited thereto, but generally, stirring may be performed at 500 to 20,000 rpm for 1 minute to 5 minutes. Solvent evaporation is performed on the homogenized emulsion, and the evaporation of the solvent may be performed while stirring at 50 to 600 RPM at a temperature of 5 to 40 °C for 1 hour to 4 hours.

In an example of the present invention, a method for preparing a microparticle by utilizing solid-state liraglutide, for example, liraglutide acetate, to mix with an O/W emulsion comprising enavogliflozin is illustrated.

An S/O/W type microparticle comprising solid-state liraglutide may be advantageous because it can suppress an excessive initial burst of liraglutide while increasing a drug encapsulation efficiency.

In another embodiment of the present invention, a depot composition comprising a microparticle comprising enavogliflozin and a microparticle comprising liraglutide is provided. In this case, when enavogliflozin is prepared into an O/W type microparticle, and liraglutide is prepared into a W/O/W or S/O/W type microparticle, it may be prepared by a method of mixing two types of microparticles. In addition, when enavogliflozin and liraglutide are each encapsulated in a microparticle by a spray drying method, a content of an active ingredient comprised in each microparticle is measured, and then an amount of a microparticle powder is quantified in consideration of an administration dose, and it is also possible to use it by mixing it for several minutes utilizing a powder lab mixer.

In the present invention, the microparticle may have an average particle size of 10 to 100 µm. Without being limited thereto, the microparticle may preferably have an average particle size of 10 to 50 µm to be suitable for use as an injection.

A preferred drug content within the entire microparticle is not limited thereto, but may be 10 to 40 wt%, and for example, comprises all sub-numerical ranges within the range, such as 10 to 20 wt%, 15 to 35 wt%, 15 to 30 wt%, 20 to 30 wt%, 20 to 27 wt%, 20 to 24 wt%, and the like.

In an embodiment of the present invention, in the case of enavogliflozin, a drug content of enavogliflozin within the microparticle may be 10 to 20 wt%, but is not limited thereto. In the case of liraglutide, a drug content of liraglutide within the microparticle may be 10 to 20 wt%, but is not limited thereto.

In the present invention, the depot composition may be for preventing or treating obesity.

Without being limited thereto, the depot composition may be subcutaneously injected once every 2 weeks to 4 weeks. It may be possible to control an administration cycle of the depot composition by controlling a drug encapsulation amount within the microparticle, a drug release rate, an amount of the depot composition injected at one time, and the like. Without being limited thereto, the depot composition may be subcutaneously injected once every 2 weeks.

Enavogliflozin is a drug currently approved as a therapeutic agent for diabetes and is sold under the product name Envlo Tablet. The dose of enavogliflozin as a therapeutic agent for diabetes was approved as 0.3 mg per day, but the dose of enavogliflozin as a therapeutic agent for obesity has not been determined. The present invention relates to an anti-obesity treatment use of enavogliflozin for combination administration with a low dose of liraglutide, and based on the following examples, the dose of enavogliflozin for combination administration together with a low dose of liraglutide may be 0.3 to 1.5 mg/day.

Here, the low-dose of liraglutide refers to a low level of dose compared to the dose of liraglutide currently used for the treatment of obesity. Liraglutide is currently approved for two uses, diabetes and obesity. While the dose for the treatment of diabetes is 0.6 to 1.8 mg daily, the dose for the treatment of obesity is 3.0 mg daily, and the approved dose for obesity treatment is much higher than the approved dose for diabetes treatment. The low-dose of liraglutide intended in the present invention means a dose of less than 3.0 mg daily, which is the current approved dose for obesity treatment, and although not limited thereto, the dose of liraglutide may be 1.2 to 2.4 mg/day. That is, when enavogliflozin and a low dose of liraglutide are administered in combination, in one embodiment, the dose of enavogliflozin may be 0.3 to 1.5 mg/day, and the dose of liraglutide may be 1.2 to 2.4 mg/day. In another embodiment, the dose of enavogliflozin may be 0.6 to 1.2 mg/day, and the dose of liraglutide may be 1.5 to 2 mg/day.

In an embodiment, the depot composition may be formulated into an injection for parenteral administration. When formulated into an injection, the sustained-release drug microparticle may be formulated into an aqueous or oily suspension through addition of a suitable excipient. For example, when the drug microparticle is formulated into a suspension, a person skilled in the art may select a dispersion medium in which the microparticle exhibits excellent dispersibility to formulate it. In addition, a preservative, an isotonic agent, and the like commonly used in a suspending agent may be added together.

In an embodiment, when the depot composition is formulated into an injection, the depot composition may exist in a vial separate from a dispersion medium, and may be prepared into a suspension immediately before administration to a patient. In an example, the present invention provides a kit comprising the depot composition, a dispersion medium, and a syringe. Alternatively, the depot composition and a suspension may be filled in a syringe, but may exist independently of each other in a separate compartment within the syringe.

### [Advantageous Effects]

The combination therapy of enavogliflozin and low-dose liraglutide provides a synergistic effect in the treatment of obesity compared to the single administration of each drug. In addition, compared to the single administration of liraglutide, the administered dose of liraglutide can be reduced, thereby reducing the direct cause of side effects related to the gastrointestinal system and cardiovascular system of liraglutide. In addition, through the combination of enavogliflozin, it has the advantage of additionally providing metabolic disease and cardiovascular disease improvement effects related to blood glucose, blood lipids, blood pressure, fatty liver, and the like.

Furthermore, the depot composition of enavogliflozin and liraglutide according to the present invention can significantly improve patient convenience and patient compliance compared to a conventional liraglutide formulation subcutaneously injected once a day, through unification of an administration route and improvement of an administration cycle.

### [Description of Drawings]

Fig. 1 is a graph showing the body weight reduction rate in an obesity-induced rat model according to the administration of the depot composition according to the present invention.
Fig. 2 is a photograph showing that the fat ratio in tissues is reduced as a body composition analysis result of an obesity-induced rat model according to the administration of the depot composition of the present invention.
Fig. 3 shows the PK analysis results of enavogliflozin according to the administration of each test substance in the test groups of G1 to G9.
Fig. 4 shows the PK analysis results of liraglutide according to the administration of each test substance in the test groups of G1 to G9.

### [Mode for Invention]

The advantages and features of the present invention, and methods for achieving them, will become clear with reference to the examples described in detail below. However, the present invention is not limited to the examples disclosed below, but will be implemented in various different forms, and these examples are merely provided to complete the disclosure of the present invention and to fully inform those of ordinary skill in the art of the scope of the invention to which the present invention belongs, and the present invention is only defined by the scope of the claims.

The inventors of the present invention conceived a preparation of a combination formulation capable of reducing an administration dose of liraglutide while co-administering it with enavogliflozin, and improving a conventional dosage regimen of the two drugs administered once a day and unification of an administration route, and evaluated anti-obesity efficacy thereof by preparing a depot composition comprising enavogliflozin and liraglutide as follows.

In the tables and drawings of the following examples, for convenience, liraglutide is also expressed by the abbreviation LIRA, and enavogliflozin is also expressed by the abbreviation ENAVO.

### [Preparation Examples 1 and 2]

As a depot composition comprising enavogliflozin and liraglutide, the depot compositions of Preparation Examples 1 and 2 were obtained according to the composition and manufacturing method of Table 1.

**[Table 1]**

| Preparation Example | Manufacturing Method | Polymer Coneentration / Type | Additives and Notes | ENAVO Concentration (w/w %) | LIRA Concentration (w/w %) | Continuous Phase(CP) Condition | 1st Homogenization Condition (RPM) | 2nd Homogenization Condition (RPM) | CP Injection Speed / Injection Temperature (°C) | Discontinuous Phase (DP) Injection Speed | Volatilization Condition Temperature / Time / RPM | Size D10/D50 /D90/D[4 ,3] |
|---|---|---|---|---|---|---|---|---|---|---|---|---|
| **1** | W/O/W | 35% PLG A RG 752H | Na Ac 1% | 16.67 | 27.17 | 0.25 % PVA 3L + 1% man nitol | 4,000 RPM 3 min | 4,000 RPM 3 min | 1.5 L/mi n 20 ° C | 5ml/ min | 25 °C / 3 hours / 150 | 3.4/26.8/ 65.1/30.9 |
| **2** | W/O/W | 30% PLA R202H | Mg( OH) 2 10 % | 16.67 | 27.17 | 0.50 % PVA 5L + 1% man nitol | 4,000 RPM 3 min | 4,000 RPM 3 min | 1.5 L/mi n 20 ° C | 5ml/ min | 30°C / 3 hours / 150 | 3.8/23.1/ 63.7/29.1 |

Liraglutide was sufficiently dissolved in primary distilled water for 30 minutes or more to prepare a first aqueous phase solution (W). Enavogliflozin was used after being sufficiently dissolved in methanol for 30 minutes or more. The biodegradable polymer was dissolved in methylene chloride, which is an oil phase solvent. An oil phase solution (O) was prepared by mixing the solution in which enavogliflozin was dissolved in methanol and the solution in which the biodegradable polymer was dissolved in methylene chloride. The first aqueous phase solution (W) and the oil phase solution (O) were mixed and homogenized using a homogenizer to prepare a W/O emulsion. In addition, an aqueous solution containing polyvinyl alcohol (PVA) as a surfactant was prepared as a second aqueous phase solution.

When the W/O emulsion is injected through a rubber pipe utilizing a micro-liquid medicine injector, microparticles are generated while being exposed to the surfactant aqueous phase solution and simultaneously mixed by a Homo-Mixer. The organic solvent present in the microparticles is removed by rotating the mixed solution of the W/O emulsion and the surfactant aqueous phase solution at a constant speed. In order to remove the organic solvent, the preparation vessel temperature was adjusted to 15 to 35°C, and maintained for 3 hours. When the removal of the organic solvent was completed, the suspension was repeatedly washed with primary distilled water to remove residual polyvinyl alcohol, and the recovered microparticles were freeze-dried.

### [Experimental Example 1] Evaluation of anti-obesity effectiveness of Preparation Examples 1 and 2

For the evaluation of the anti-obesity effectiveness of the depot compositions of Preparation Examples 1 and 2, obesity was induced in 5-week-old male SD rats to be used as an animal model.

Obesity was induced by feeding a high-fat diet to rats for 13 weeks. To minimize the stress received by the experimental animals due to subcutaneous administration of the test substance, physiological saline was subcutaneously administered to all animals from 2 weeks before the administration of the test substance to perform pre-adaptation training for subcutaneous administration.

The test groups were set as a vehicle group (G1), an enavogliflozin single administration group (G2), a Saxenda^{™} (high-dose liraglutide) single administration group (G3), and groups administered every 2 or 4 weeks with a depot formulation in which enavogliflozin and low-dose liraglutide were encapsulated (G4 to G6) as follows (n=8 per each group). All test groups were administered with the test substance for 4 weeks through subcutaneous injection (S.C) of a 2 mg/kg volume including the test substance and vehicle.

**[Table 2]**

| Gro up | Group Name | Test Substance and Single Dose | Dosing Regimen |
|---|---|---|---|
| G1 | Vehicle | Vehicle (PBS) | Once a day |
| G2 | Enavogliflozin | Enavogliflozin (0.30 mg/kg) | Once a day |
| G3 | Saxenda^{™} | Liraglutide (0.31 mg/kg) | Once a day |
| G4 | Preparation Example 1(2W) | ENAVO (5.32mg/kg), LIRA(2.17mg/kg) | once every 2 weeks |
| G5 | Preparation Example 2(2W) | ENAVO (4.20mg/kg), LIRA(2.17mg/kg) | once every 2 weeks |
| G6 | Preparation Example 2(4W) | ENAVO (8.40mg/kg), LIRA(4.34mg/kg) | once every 4 weeks |

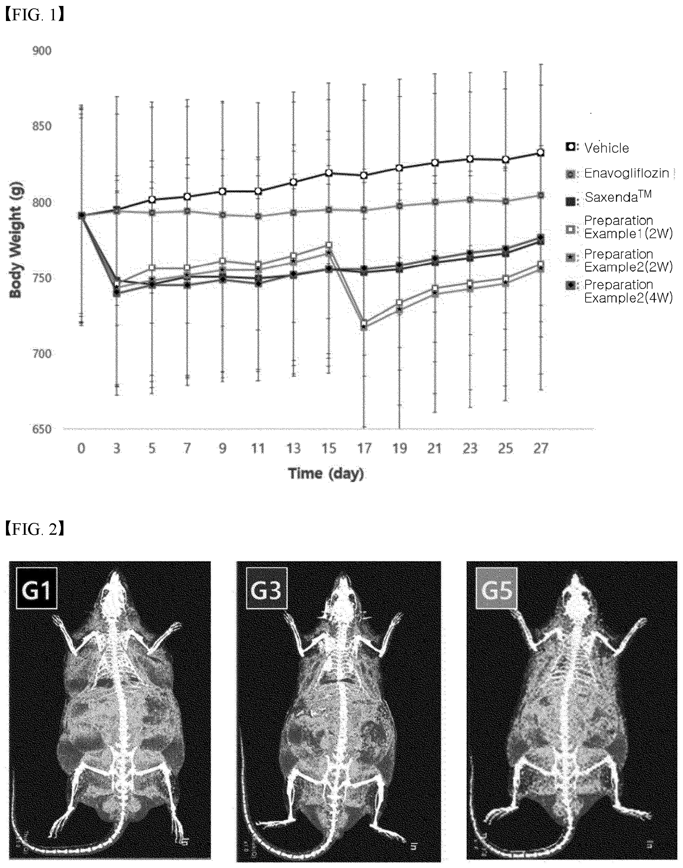

As a result of analyzing the body weight reduction rate for each group, as can be seen in Fig. 1, the body weight reduction effect of G2 compared to G1 was minimal (-3.39%), but the case of G4 (-8.85%) and G5 (-9.26%), which are the combination depot injection groups combined with low-dose liraglutide administered at 2-week intervals, showed superior body weight reduction effect than G3 (-7.09%), which is the high-dose liraglutide single administration group. In addition, the case of G6 (-6.75%), which is the combination depot injection group combined with low-dose liraglutide administered at 4-week intervals, showed a body weight reduction effect similar to G3 (-7.09%), which is the high-dose liraglutide single administration group. In the case of G6, compared to G3, which must be administered 28 times over 4 weeks, it is a formulation in which patient medication convenience is significantly improved in that it is a body weight reduction effect obtained through only a single injection administration.

Furthermore, as a result of analyzing the fat weight and the proportion of fat within tissues through body composition analysis, as presented in Table 3 and Fig. 2, the depot injection administration group G5 comprising enavogliflozin and low-dose liraglutide exhibited a reduction in fat mass similar to G3, which is the high-dose liraglutide single administration group, compared to the vehicle group (the red areas in Fig. 2 represent fat).

**[Table 3]**

| Body fat mass and muscle mass in SD rats fed 60% HFD during the experimental period while receiving test substances | | | | | | | | | | |
|---|---|---|---|---|---|---|---|---|---|---|
| Group | | | Value (g) | | | | BMD (g/cm ²) | Bone Area (cm²) | Bone Volu me (cm³) | FAT In Tissue (%) |
| | | | Total Mass | BMC | FAT | LEAN | | | | |
| G1 | N | Mean | 856.16 | 19.4574 | 358.8684 | 477.8379 | 0.2446 | 79.5694 | 11.7686 | 42.6893 |
| Vehicle | 8 | SD | 65.28 | 1.0066 | 58.8570 | 28.7493 | 0.0095 | 3.4683 | 0.6088 | 4.3588 |
| G2 | N | Mean | 820.03 | 18.8277 | 311.1294 | 490.0705 | 0.2423 | 77.7263 | 11.3877 | 38.6281 |
| Enavogli flozin | 8 | SD | 73.05 | 1.0777 | 52.6437 | 30.3652 | 0.0059 | 4.4565 | 0.6518 | 3.6568 |
| G3 | N | Mean | 793.24 | 19.1914 | 293.3549 | 480.6909 | 0.2425 | 79.0754 | 11.6077 | 37.7066 |
| Saxenda^{™} | 8 | SD | 65.20 | 1.6238 | 47.8188 | 26.4159 | 0.0092 | 4.8024 | 0.9821 | 3.5796 |
| G4 | N | Mean | 778.91 | 18.5477 | 304.1375 | 456.2213 | 0.2350 | 78.9468 | 11.2184 | 39.7680 |
| Preparati on Example 1(2W) | 7 | SD | 79.42 | 1.4308 | 54.6773 | 32.0108 | 0.0074 | 5.7322 | 0.8654 | 3.4135 |
| G5 | N | Mean | 775.81 | 18.6810 | 289.3078 | 467.8231 | 0.2391 | 78.1144 | 11.2990 | 37.9376 |
| Preparation Example 2(2W) | 8 | SD | 72.93 | 0.9111 | 54.3308 | 29.1760 | 0.00464 | 3.0489 | 0.5510 | 4.2511 |
| G6 | N | Mean | 794.12 | 18.4799 | 297.5711 | 478.0734 | 0.2340* | 79.0588 | 11.1774 | 38.1964 |
| Preparation Example 2(4W) | 8 | SD | 55.28 | 0.5802 | 46.4074 | 26.7088 | 0.0095 | 3.4453 | 0.3509 | 4.0630 |

BMC: bone mineral content; BMD: bone mineral density. Significant difference from G1 HFD vehicle control group by One-way ANOVA followed by the Dunnett's test: * p<0.05, ** p<0.01.

In addition, as a result of serological analysis, as can be seen in Table 4, the LDL-C reduction result was significantly reduced in G4, which is the combination depot injection group comprising enavogliflozin and low-dose liraglutide, compared to the result of G3, which is the high-dose liraglutide single administration group. In addition, in G5 and G6, which are the combination depot injection groups comprising enavogliflozin and low-dose liraglutide, the TG level was also significantly reduced compared to the G1 group.

Furthermore, in the blood chemistry analysis, as shown in Table 8, the reduction in LDL-C was significantly greater in the combination depot group G4 comprising enavogliflozin and low-dose liraglutide compared to the result of the high-dose liraglutide single administration group G3. Moreover, in the combination depot groups G5 and G6 comprising enavogliflozin and low-dose liraglutide, TG levels were also significantly reduced compared to the G1 group.

**[Table 4] Blood chemistry analysis results of SD rats fed 60% HFD during the experimental period while receiving test substances**

| Group | | | Blood chemistry analysis | | | | | |
|---|---|---|---|---|---|---|---|---|
| | | | AST (U/L) | ALT (U/L) | TG (mg/dL) | T-Chol (mg/dL) | LDL-C (mg/dL) | HDL-C (mg/dL) |
| G1 | N | Mean | 339.63 | 198.13 | 79.25 | 96.75 | 6.88 | 22.63 |
| Vehicle | 8 | SD | 306.04 | 180.52 | 21.83 | 21.17 | 1.55 | 4.84 |
| G2 | N | Mean | 226.50 | 123.38 | 61.13 | 86.38 | 5.13 | 24.00 |
| Enavogliflozin | 8 | SD | 151.19 | 96.09 | 18.70 | 18.57 | 0.64 | 3.25 |
| G3 | N | Mean | 184.25 | 105.50 | 40.88** | 80.50 | 6.13 | 22.88 |
| Saxenda^{®} | 8 | SD | 84.12 | 99.47 | 10.26 | 15.72 | 0.99 | 4.64 |
| G4 | N | Mean | 167.29 | 72.86 | 52.71 | 78.86 | 4.71* | 22.57 |
| Preparation Example 1(2W) | 7 | SD | 57.83 | 38.37 | 12.20 | 24.41 | 0.95 | 6.05 |
| G5 | N | Mean | 157.75 | 73.63 | 49.63* | 74.38 | 5.00 | 22.25 |
| Preparation Example | 8 | SD | 79.28 | 43.23 | 10.93 | 16.07 | 1.41 | 3.96 |
| 2(2W) | | | | | | | | |
| G6 | N | Mean | 135.88 | 55.75 | 51.50* | 90.75 | 6.75 | 21.88 |
| Preparation Example 2(4W) | 8 | SD | 42.83 | 27.14 | 3.89 | 12.16 | 1.83 | 2.17 |

Significant difference from G1 vehicle control group by One-way ANOVA followed by the Dunnett's test: * p<0.05, ** p<0.01.
AST: aspartate aminotransferase, ALT: alanine aminotransferase, TG: triglyceride T-Chol: total cholesterol, LDL-C: low density lipoprotein-cholesterol, HDL-C: high density lipoprotein-cholesterol.

Furthermore, as shown in Table 5, which compares the internal organ weights of SD rats at necropsy after the end of the study, the absolute liver weight was significantly reduced in the combination depot groups G4, G5, and G6 comprising enavogliflozin and low-dose liraglutide compared to the result of the high-dose liraglutide single administration group G3. In addition, in the combination depot group G6, the relative organ weight of the liver was also significantly reduced compared to the G1 group.

**[Table 5]**

| Absolute organ weight (g) and relative organ weight (organ weight/body weight ratio) at sacrifice | | | | | | | |
|---|---|---|---|---|---|---|---|
| Group | | | Body weight (g) | Absolute organ weight (g) | | Relative organ weight | |
| | | | | liver | epididymal fat | liver | epididymal fat |
| G1 | N | Mean | 811.13 | 19.5015 | 26.1435 | 0.0240 | 0.0321 |
| Vehicle | 8 | SD | 60.05 | 3.0972 | 4.4436 | 0.0030 | 0.0044 |
| G2 | N | Mean | 777.17 | 18.6817 | 23.2977 | 0.0241 | 0.0295 |
| Enavogliflozin | 8 | SD | 71.98 | 2.8227 | 7.0975 | 0.0032 | 0.0065 |
| G3 | N | Mean | 744.09 | 17.1632 | 21.5237 | 0.0231 | 0.0287 |
| Saxenda^{®} | 8 | SD | 62.64 | 2.3700 | 4.5815 | 0.0027 | 0.0041 |
| G4 | N | Mean | 729.42 | 15.7746* | 21.6260 | 0.0218 | 0.0296 |
| Preparation Example 1(2W) | 7 | SD | 75.52 | 1.4751 | 3.9017 | 0.0025 | 0.0042 |
| G5 | N | Mean | 735.46 | 15.2485* | 20.6418 | 0.0206 | 0.0281 |
| Preparation Example 2(2W) | 8 | SD | 69.09 | 2.7544 | 3.2107 | 0.0024 | 0.0041 |
| G6 | N | Mean | 751.34 | 14.5738* * | 23.4828 | 0.0194** | 0.0311 |
| Preparation Example 2(4W) | 8 | SD | 52.72 | 1.1108 | 6.1003 | 0.0013 | 0.0071 |

Significant difference from G1 HFD vehicle control group by One-way ANOVA followed by the Dunnett's test: * p<0.05, ** p<0.01.

In conclusion, in a rat model of obesity induced by 13 weeks of high-fat diet feeding, the combination depot formulations G4 and G5 comprising enavogliflozin and low-dose liraglutide administered once every 2 weeks demonstrated superior body weight reduction effects compared to the existing obesity treatment Saxenda^{™} (high-dose liraglutide administration) group G3. The 4-week interval combination depot G6 also showed a body weight reduction effect similar to G3. Furthermore, comprehensive analysis of fat weight, blood chemistry analysis, and absolute/relative liver weight results confirmed that the combination depot administration comprising enavogliflozin and low-dose liraglutide provides additional benefits in improving obesity-related metabolism that cannot be confirmed with high-dose liraglutide alone, while simultaneously reducing side effects, achieving comparable body weight loss, and furthermore, improving patient medication convenience.

Accordingly, further studies related to the sustained drug release of the depot composition were continued in the subsequent research.

### [Preparation Examples 3 to 10]

In order to secure sustained drug release of enavogliflozin and liraglutide, depot compositions comprising enavogliflozin and liraglutide were prepared through various attempts (S/O/W preparation method, spray drying preparation method, and a post-mixing preparation method where enavogliflozin and liraglutide were separately encapsulated into microparticles, recovered by freeze-drying, and then mixed). The specific manufacturing method is as follows.

In Preparation Examples 3 to 10, enavogliflozin and liraglutide acetate were used as active ingredients. They were manufactured using the S/O/W manufacturing method, the Spray drying manufacturing method, or a method of separately encapsulating each active ingredient in microparticles and then post-mixing them.

First, to prepare liraglutide acetate utilizing hydrophobic ion pairing, 10 g of liraglutide and 4.2 g of sodium acetate were dissolved in 400 ml of primary distilled water and mixed for 30 minutes. To the mixture, 10 ml of acetic acid (Glacial, Sigma) or 10 ml of 1N HCl was added to lower the pH, thereby precipitating liraglutide acetate, and the mixture was allowed to further react for 4 hours. Thereafter, it was repeatedly washed with primary distilled water and methanol, filtered through a 635 Mesh sieve, and the recovered liraglutide acetate was recovered by freeze-drying.

In the case of Preparation Example 3, a PLA polymer of R202H was dissolved in methylene chloride at a weight ratio of 30 w/w% and used, and enavogliflozin was completely dissolved in methanol at a weight ratio of 16.7 w/w%. Then, the polymer solution and solid-phase liraglutide acetate were mixed using a Homo-mixer at 10,000 RPM for 3 minutes to prepare a first mixed solution (S/O emulsion). Thereafter, the first mixed solution (S/O emulsion) was introduced into a 5L water bath containing a surfactant (PVA) at a weight ratio of 0.5%, and simultaneously a Homo-mixer was applied at 4,500 RPM for 3 minutes, thereby preparing a second mixed solution (S/O/W microparticles). For the solvent evaporation process, the water bath temperature was adjusted to 30°C, and the mixed solution was stirred at 150 RPM using an overhead stirrer for 3 hours. Thereafter, microparticles were recovered through centrifugation at 1,500 to 3,000 RPM for 3 minutes, and washed 3 times repeatedly using primary distilled water to wash away unencapsulated active ingredients and the surfactant. Thereafter, freeze-drying was performed at - 40°C to 5°C for 48 hours, thereby recovering microparticle powder. The recovered microparticles were passed through a 200 Mesh sieve, thereby finally securing the microparticles.

In the case of Preparation Example 4, a PLA polymer of R203H was dissolved in methylene chloride at a weight ratio of 23 w/w% and used, and enavogliflozin was completely dissolved in methanol at a weight ratio of 16.7 w/w%. Then, the polymer solution and solid-phase liraglutide acetate were mixed using a Homo-mixer at 10,000 RPM for 3 minutes to prepare a first mixed solution (S/O emulsion). Thereafter, the first mixed solution (S/O emulsion) was introduced into a 1L water bath containing a surfactant (PVA, Gohsenol EG-40PW) at a weight ratio of 1.0 w/w% while applying a Homo-mixer at 3,500 RPM for 3 minutes, thereby preparing a second mixed solution (S/O/W microparticles). For the solvent evaporation process, the water bath temperature was adjusted to 30°C, and the solvent evaporation process was carried out at 150 RPM using an overhead stirrer for 3 hours. Thereafter, microparticles were recovered through centrifugation at 1,500 to 3,000 RPM for 3 minutes, and washed 3 times repeatedly using primary distilled water to wash away unencapsulated active ingredients and the surfactant. Thereafter, freeze-drying was performed at -40°C to 5°C for 48 hours, thereby recovering microparticle powder. The recovered microparticles were passed through a 200 Mesh sieve, thereby finally securing the microparticles.

In the case of Preparation Example 5, a PLA polymer of R203H was dissolved in methylene chloride at a weight ratio of 15 w/w% and used, and enavogliflozin was completely dissolved in methanol at a weight ratio of 16.7 w/w%. Then, the polymer solution and solid-phase liraglutide acetate were mixed using a Homo-mixer at 15,000 RPM for 3 minutes to make a mixed solution (S/O emulsion), and then microparticles were prepared using a Spray dryer (BUCHI, Mini B-290). Here, the mixed solution was injected at a speed of 1.0 ml/min using an injection pump, the Inlet temperature was set to 55 to 75°C, the Outlet temperature was set to 36 to 42°C, and the mixed solution was atomized at a spray pressure of 20 to 50 psi, thereby manufacturing and recovering microparticles.

In the case of Preparation Example 6, a PLA polymer of R203H was dissolved in methylene chloride at a weight ratio of 23 w/w% and used, and enavogliflozin was completely dissolved in methanol at a weight ratio of 16.7 w/w%. Then, the polymer solution and solid-phase liraglutide acetate were mixed using a Homo-mixer at 10,000 RPM for 3 minutes to prepare a first mixed solution (S/O emulsion). Thereafter, the first mixed solution (S/O emulsion) was introduced into a 1L water bath containing a surfactant (PVA, Merck 05P) at a weight ratio of 1.0%, and simultaneously a Homo-mixer was applied at 3,500 RPM for 3 minutes, thereby preparing a second mixed solution (S/O/W microparticles). For the solvent evaporation process, the water bath temperature was adjusted to 35°C, and a solvent evaporation process was performed at 150 RPM using an overhead stirrer for 3 hours. Thereafter, microparticles were recovered through centrifugation at 1,500 to 3,000 RPM for 3 minutes, and washed 3 times repeatedly using primary distilled water to wash away unencapsulated active ingredients and the surfactant. Thereafter, freeze-drying was performed at -40°C to 5°C for 48 hours, thereby recovering microparticle powder. The recovered microparticles were passed through a 200 Mesh sieve, thereby finally securing the microparticles.

In the case of Preparation Example 7, enavogliflozin and liraglutide acetate were separately manufactured without being mixed. For manufacturing enavogliflozin microparticles, a PLA polymer of R203H was dissolved in methylene chloride at a weight ratio of 23 w/w% and used, and after completely dissolving enavogliflozin in methanol at a weight ratio of 16.7 w/w%, the polymer solution was mixed using a Homo-mixer at 10,000 RPM for 3 minutes to prepare a first mixed solution (S/O emulsion). Thereafter, the first mixed solution (S/O emulsion) was introduced into a 1L water bath containing a surfactant (PVA, Merck 05P) at a weight ratio of 1.0 w/w% while applying a Homo-mixer at 3,500 RPM for 3 minutes, thereby preparing a second mixed solution (S/O/W microparticles). For the solvent evaporation process, the water bath temperature was adjusted to 30°C, and the solvent evaporation process was carried out at 150 RPM using an overhead stirrer for 3 hours. Thereafter, microparticles were recovered through centrifugation at 1,500 to 3,000 RPM for 3 minutes, and washed 3 times repeatedly using primary distilled water to wash away unencapsulated active ingredients and the surfactant. Thereafter, freeze-drying was performed at -40°C to 5°C for 48 hours to recover microparticle powder. The recovered microparticles were passed through a 200 Mesh sieve, thereby finally securing the microparticles. In addition, for manufacturing liraglutide acetate microparticles, a PLA polymer of R203H was dissolved in methylene chloride at a weight ratio of 23 w/w% and used, and other manufacturing processes were carried out identically to the microparticle manufacturing conditions in which enavogliflozin was separately encapsulated as described above.

In the case of Preparation Example 8, a PLA polymer of R202H was dissolved in methylene chloride at a weight ratio of 30 w/w% and used, and after completely dissolving enavogliflozin in methanol at a weight ratio of 16.7 w/w%, the polymer solution and solid-phase liraglutide acetate were mixed using a Homo-mixer at 15,000 RPM for 3 minutes to prepare a first mixed solution (S/O emulsion).

Thereafter, the first mixed solution (S/O emulsion) was introduced into a 1L water bath containing a surfactant (PVA, Merck 05P) at a weight ratio of 1.0 w/w% while applying a Homo-mixer at 3,500 RPM for 3 minutes, thereby preparing a second mixed solution (S/O/W microparticles). For the solvent evaporation process, the water bath temperature was adjusted to 30°C, and a solvent evaporation process was performed at 150 RPM using an overhead stirrer for 3 hours. Thereafter, microparticles were recovered through centrifugation at 1,500 to 3,000 RPM for 3 minutes, and the recovered microparticles were washed once utilizing PBST (Tween 80, 0.05%) and then washed 3 times repeatedly utilizing primary distilled water to wash away unencapsulated active ingredients and the surfactant. Thereafter, freeze-drying was performed at - 40°C to 5°C for 48 hours, thereby recovering microparticle powder. The recovered microparticles were passed through a 200 Mesh sieve, thereby finally securing the microparticles.

In the case of Preparation Example 9, a PLA polymer of R203H was dissolved in methylene chloride at a weight ratio of 23 w/w% and used. After completely dissolving enavogliflozin in methanol at a weight ratio of 16.7 w/w%, 1N HCl instead of acetic acid was added to the mixture to lower the pH, thereby precipitating liraglutide acetate. Then, the polymer solution and solid-phase liraglutide acetate were mixed using a Homo-mixer at 15,000 RPM for 2.5 minutes to prepare a first mixed solution (S/O emulsion). Thereafter, the first mixed solution (S/O emulsion) was introduced into a 1L water bath containing a surfactant (PVA, Merck 05P) at a weight ratio of 1.0% while applying a Homo-mixer at 3,500 RPM for 3 minutes, thereby preparing a second mixed solution (S/O/W microparticles). For the solvent evaporation process, the water bath temperature was adjusted to 30°C, and the solvent evaporation process was carried out at 150 RPM using an overhead stirrer for 3 hours. Thereafter, microparticles were recovered through centrifugation at 1,500 to 3,000 RPM for 3 minutes, and the recovered microparticles were washed once utilizing PBST (Tween 80, 0.05%) and then washed 3 times repeatedly utilizing primary distilled water to wash away unencapsulated active ingredients and the surfactant. Thereafter, freeze-drying was performed at -40°C to 5°C for 48 hours to recover microparticle powder. The recovered microparticles were passed through a 200 Mesh sieve, thereby finally securing the microparticles.

In the case of Preparation Example 10, a PLA polymer of R203H was dissolved in methylene chloride at a weight ratio of 23 w/w% and used. After completely dissolving enavogliflozin in methanol at a weight ratio of 16.7 w/w%, the polymer solution and solid-phase liraglutide acetate were mixed.

In the case of Preparation Example 10, a PLA polymer of R203H was dissolved in methylene chloride at a weight ratio of 23% and used. After completely dissolving enavogliflozin in methanol at a weight ratio of 16.7%, the polymer solution and the solid-phase liraglutide acetate-which was secured through a recovery process after precipitation by repeatedly washing with primary distilled water and completing the final washing utilizing methanol-were mixed. Then, the mixture was mixed using a Homo-mixer at 10,000 RPM for 3 minutes to prepare a first mixed solution (S/O emulsion). Thereafter, the first mixed solution (S/O emulsion) was introduced into a 1L water bath containing a surfactant (PVA, Merck 05P) at a weight ratio of 1.0%, and simultaneously a Homo-mixer was applied at 3,500 RPM for 3 minutes to prepare a second mixed solution (S/O/W microparticles). For the solvent evaporation process, the water bath temperature was adjusted to 30°C, and the mixed solution was stirred at 150 RPM using an overhead stirrer for 3 hours. Thereafter, microparticles were recovered through centrifugation at 1,500 to 3,000 RPM for 3 minutes, and washed once utilizing PBST (Tween 80, 0.05%) and then washed 3 times repeatedly utilizing primary distilled water to wash away unencapsulated active ingredients and the surfactant. Thereafter, freeze-drying was performed at -40°C to 5°C for 48 hours to recover microparticle powder. The recovered microparticles were passed through a 200 Mesh sieve, thereby finally securing the microparticles.

In order to check the diameter and particle size distribution of the combination microparticles containing enavogliflozin and liraglutide acetate, analysis was performed using a Malvern particle size analyzer.

Specifically, the depot compositions of Preparation Examples 3 to 10 were obtained according to the composition and manufacturing method of Table 6.

**[Table 6]**

| **Prepa ration Exam ple** | **Preparat ion Method** | **Pol yme r Con cent rati on & Typ e** | **Addit ive & Speci al Featu res** | **ENAV O Conce ntrati on w/w %** | **Continuo us Phase Conditio n** | **1st Ho mog eniz atio n Con diti on (RP M)** | **2nd Ho mog eniz atio n Con diti on (RP M)** | **Solvent Evapor ation Conditi on (Temp/ Time/R PM)** | **Particle Size D10/D50/D 90/D[4,3]** |
|---|---|---|---|---|---|---|---|---|---|
| **3** | S/O/W | 30% PL A R20 2H | LIRA Aceta te | 16.7 | 0.50% PVA 5L Gohsenol EG-40PW | 10,0 00 3 min | 4,50 0 3 min | 30°C / 3 / 150 | 2.3/13.0/37. 4/**16.9** |
| **4** | S/O/W | 23% PL A R20 3H | LIRA Aceta te | 16.7 | 1.0%, PVA 1L Gohsenol EG-40PW | 10,0 00 3 min | 3,50 0 3 min | 30°C / 3 / 150 | 1.9/13.0/34. 2/**15.7** |
| **5** | Spray Drying | 15% PL A R20 3H | LIRA Aceta te | 16.7 | 1.0%, PVA 1L Merck PVA 05P | 15,0 00 3 min | N.A | N.A | 2.7/17.6/44. 2/**21.6** |
| **6** | S/O/W | 23% PL A R20 3H | LIRA Aceta te | 16.7 | 1.0%, PVA 1L Merck PVA 05P | 10,0 00 3 min | 3,50 0 3 min | 35°C / 3 / 150 | 2.0/15.6/36. 4/**17.5** |
| **7** | O/W | 23% PL A R20 2H | ENA VO separ ately prepa red | 16.7 | 1.0%, PVA 1L Merck PVA 05P | 10,0 00 3 min | 3,50 0 3 min | 30°C / 3 / 150 | 2.0/14.6/33. 3/**18.1** |
| | S/O/W | 23% PL A R20 3H | LIRA - Aceta te | N.A | 1.0%, PVA 1L Merck PVA 05P | 10,0 00 3 min | 3,50 0 3 min | 30°C / 3 / 150 | 2.0/16.6/37. 6/**22.2** |
| **8** | S/O/W | 30% PL A R20 2H | LIRA - Aceta te /PBS T 0.05 % washi ng | 16.7 | 1.0%, PVA 1L Merck PVA 05P | 15,0 00 3 min | 3,50 0 3 min | 30°C / 3 / 150 | 8.0/31**.**2/64. 0/**34.0** |
| **9** | S/O/W | 23% PL A R20 3H | LIRA - Aceta te (HCl) | 16.7 | 1.0%, PVA 1L Merck PVA 05P | 15,0 00 2.5 min | 3,50 0 3 min | 30°C / 3 / 150 | 5.2/24**.**4/51. 2/**26.9** |
| **10** | S/O/W | 30% PL A R20 2H | LIRA - Aceta te/ MeO H Wash ing | 16.7 | 1.0%, PVA 1L Merck PVA 05P | 10,0 00 3 min | 3,50 0 3 min | 30°C / 3 / 150 | 7.4/28**.**8/65. 1/**32.1** |

### [Experimental Example 2] PK analysis according to administration of depot compositions of Preparation Examples 3 to 10

For PK analysis according to the administration of the depot compositions of Preparation Examples 3 to 10, 5-week-old male SD rats were used as an animal model.

As shown in Table 7, the test groups were set as an enavogliflozin + liraglutide combination administration group (G1) and the depot formulation administration groups of

Preparation Examples 3 to 10 in which enavogliflozin and liraglutide were encapsulated (G2 to G9) as follows (G1: n=3, G2 to G9: n=2 per each group).

**[Table 7]**

| Test Substance | | Dosing Regimen | Dose (mg/kg) | Volume (mL/kg) |
|---|---|---|---|---|
| G1 | enavogliflozin + liraglutide | SC once daily | enavogliflozin 0.093+ liraglutide 0.186 | enavogliflozin 0.93+ liraglutide 1.86 |
| G2 | Preparation Example 3 | | enavogliflozin 1.87 + liraglutide 2.60 | |
| G3 | Preparation Example 4 | | enavogliflozin 2.86 + liraglutide 2.60 | |
| G4 | Preparation Example 5 | | enavogliflozin 2.77 + liraglutide 2.60 | |
| G5 | Preparation Example 6 | SC single administr ation (single dose) | enavogliflozin 3.00 + liraglutide 2.60 | 1.2 |
| G6 | Preparation Example 7 | | enavogliflozin 2.60 + liraglutide 2.60 | |
| G7 | Preparation Example 8 | | enavogliflozin 2.30 + liraglutide 2.60 | |
| G8 | Preparation Example 9 | | enavogliflozin 1.68 + liraglutide 2.60 | |
| G9 | Preparation Example 10 | | enavogliflozin 1.86 + liraglutide 2.60 | |

Plasma samples were collected at designated times, and samples were stored at -70°C until analyzed using an LC-MS/MS system. Protein precipitation was performed on 20 µL of plasma samples using 180 µL of acetonitrile containing an internal standard. After mixing and centrifugation, the supernatant (150 µL) was analyzed with an LC-MS/MS system equipped with an MRM mode. LC/MS/MS data were analyzed by Analyst 1.6.3. PK parameters were calculated by non-compartmental analysis using WinNonlin software.

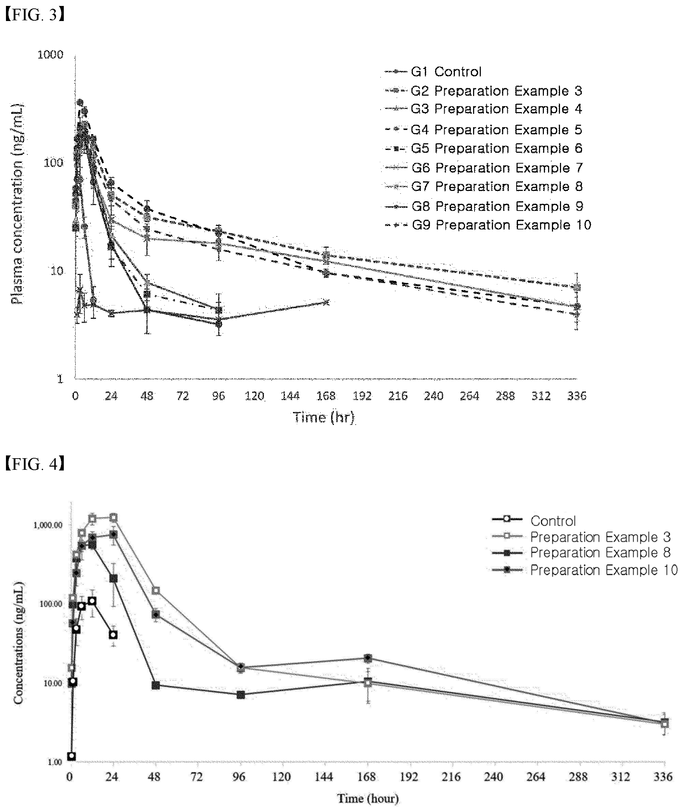

Fig. 3 shows the PK analysis results of enavogliflozin according to the administration of each test substance.

The sustained drug release level of the drug was evaluated through the plasma drug concentrations measured at 4 weeks (a total of 672 hours) after administration of the test substances. As a result, compared to the ENAVO control drug administration group (G1), the drug was detected at the 2-week time point (336 h) in groups G2, G4, G7, and G9. In addition, compared to the ENAVO control drug administration group (G1), the drug was detected up to the 4-week time point (672 h) in groups G7 and G9, confirming that the drug was continuously released.

Figure 4 shows the PK analysis results of liraglutide according to the administration of each test substance.

The sustained drug release level was evaluated through the plasma drug concentrations measured at 4 weeks (a total of 672 hours) after administration of the test substances. As a result, compared to the LIRA control group (G1), drug was detected at the 2-week time point (336 h) in groups G2, G7, and G9, confirming that sustained drug release was achieved.

In summary, in the case of the depot compositions of Preparation Example 3, Preparation Example 8, and Preparation Example 10, it was confirmed that both enavogliflozin and liraglutide exhibited sustained drug release for more than 2 weeks.

More preferably, it was confirmed that the liraglutide drug release from the depot composition of Preparation Example 10 was maintained at a relatively higher level at the 2-week time point (336 h) compared to the other Preparation Examples.

## Claims

1. A depot composition comprising microparticles comprising enavogliflozin and liraglutide as active ingredients, and a biodegradable polymer.

2. The depot composition according to claim 1, wherein the biodegradable polymer is selected from the group consisting of polylactide (PLA), polyglycolide (PGA), poly(lactide-co-glycolide) (PLGA), and a mixture thereof.

3. The depot composition according to claim 1, wherein the biodegradable polymer has a weight average molecular weight of 4,000 to 50,000.

4. The depot composition according to claim 1, wherein the microparticles are prepared by a solvent evaporation method or a spray drying method.

5. The depot composition according to claim 1, wherein the microparticles are W/O/W type microparticles.

6. The depot composition according to claim 5, wherein the W/O/W type microparticles comprise sodium acetate or magnesium hydroxide as an additive during preparation of a W/O emulsion.

7. The depot composition according to claim 1, wherein the microparticles are S/O/W type microparticles.

8. The depot composition according to claim 7, wherein the liraglutide in the S/O/W type microparticles is in the form of solid-state liraglutide acetate.

9. The depot composition according to claim 1, wherein the microparticles have an average particle size of 10 to 100 µm.

10. The depot composition according to claim 1, wherein a content of enavogliflozin and a content of liraglutide in the microparticles are each 10 to 20 wt%.

11. The depot composition according to claim 1, wherein the depot composition is for use in prevention or treatment of obesity.

12. The depot composition according to claim 1, wherein the depot composition is formulated for subcutaneous injection once every 2 to 4 weeks.

13. The depot composition according to claim 1, wherein the depot composition is formulated to administer enavogliflozin at a dose of 0.3 to 1.5 mg/day and liraglutide at a dose of 1.2 to 2.4 mg/day.

14. The depot composition according to claim 1, wherein the depot composition is formulated to administer enavogliflozin at a dose of 0.6 to 1.2 mg/day and liraglutide at a dose of 1.5 to 2 mg/day.

15. A kit comprising the depot composition according to any one of claims 1 to 14, a dispersion medium, and a syringe.
